# EUROPEAN PATENT SPECIFICATION

(11) **EP 3 415 628 B1**
(45) Date of publication and mention of the grant of the patent: **08.03.2023**
(21) Application number: 17738613.3
(22) Date of filing: 10.01.2017
(51) Int. Cl.: C12N 15/63, C12N 9/02, C12P 7/46

(54) **RECOMBINANT MUTANT MICROORGANISM HAVING MALONIC ACID PRODUCTION CAPABILITY AND METHOD FOR PRODUCING MALONIC ACID USING SAME**
REKOMBINANTER MUTANTER MIKROORGANISMUS MIT MALONSÄUREPRODUKTIONSFÄHIGKEIT UND VERFAHREN ZUR HERSTELLUNG VON MALONSÄURE UNTER VERWENDUNG DAVON
MICRO-ORGANISME MUTANT RECOMBINANT PRÉSENTANT UNE CAPACITÉ DE PRODUCTION D'ACIDE MALONIQUE ET PROCÉDÉ DE PRODUCTION D'ACIDE MALONIQUE L'UTILISANT

(30) Priority: 11.01.2016 KR 20160003228
(43) Date of publication of application: 19.12.2018
(73) Proprietor: Korea Advanced Institute of Science and Technology, Daejeon 34141 (KR); Hanwha Chemical Corporation, Seoul 04541 (KR)
(72) Inventor: LEE, Sang Yup, Daejeon 34049 (KR); SONG, Chan Woo, Daejeon 34141 (KR); KO, Yoosung, Daejeon 34141 (KR); KIM, Je Woong, Daejeon 34141 (KR)
(74) Representative: dompatent von Kreisler Selting Werner - Partnerschaft von Patent- und Rechtsanwälten mbB
(86) International application number: PCT/KR2017/000319
(87) International publication number: WO 2017/122984

(56) References cited:
- WO-A1-2013/134424
- JP-A- 2015 531 237
- KR-A- 20080 069 678
- KR-A- 20100 015 743
- KR-A- 20120 103 958
- KR-A- 20120 108 538
- CHAN WOO SONG ET AL: "Metabolic Engineering of Escherichia coli for the Production of 3-Hydroxypropionic Acid and Malonic Acid through &bgr;-Alanine Route", ACS SYNTHETIC BIOLOGY, 14 March 2016 (2016-03-14), pages 1256-1263, XP055398757, Washington, DC,USA ISSN: 2161-5063, DOI: 10.1021/acssynbio.6b00007
- NAKAMURA K ET AL: "Studies of malonic semialdehyde dehydrogenase from Pseudomonas aeruginosa", BIOCHIMICA ET BIOPHYSICA ACTA, ELSEVIER, NL, vol. 50, no. 1, 10 June 1961 (1961-06-10), pages 147-152, XP023560168, ISSN: 0006-3002, DOI: 10.1016/0006-3002(61)91071-X [retrieved on 1961-06-10]
- CHAN WOO SONG ET AL: "Metabolic engineering of Escherichia coli for the production of 3-aminopropionic acid", METABOLIC ENGINEERING, vol. 30, 7 June 2015 (2015-06-07), pages 121-129, XP055586351, US ISSN: 1096-7176, DOI: 10.1016/j.ymben.2015.05.005
- VINOD KUMAR ET AL: "Recent advances in biological production of 3-hydroxypropionic acid", BIOTECHNOLOGY ADVANCES, vol. 31, no. 6, 1 November 2013 (2013-11-01), pages 945-961, XP055093428, ISSN: 0734-9750, DOI: 10.1016/j.biotechadv.2013.02.008
- T. FUHRER ET AL: "Computational Prediction and Experimental Verification of the Gene Encoding the NAD+/NADP+-Dependent Succinate Semialdehyde Dehydrogenase in Escherichia coli", JOURNAL OF BACTERIOLOGY, vol. 189, no. 22, 15 November 2007 (2007-11-15), pages 8073-8078, XP055590640, US ISSN: 0021-9193, DOI: 10.1128/JB.01027-07
- SOL CHOI ET AL: "Biorefineries for the production of top building block chemicals and their derivatives", METABOLIC ENGINEERING, vol. 28, 7 January 2015 (2015-01-07), pages 223-239, XP055332693, US ISSN: 1096-7176, DOI: 10.1016/j.ymben.2014.12.007
- SONG ET AL.: 'Metabolic Engineering of Eschehchia Coli for the Production of 3-hydroxypropionic Acid and Malonic Acid through beta-alanine Route' ACS SYNTHETIC BIOLOGY vol. 5, 29 February 2016, pages 1256 - 1263, XP055398757
- CHUTRAKUL CHANIKUL ET AL: "Metabolic engineering of long chain-polyunsaturated fatty acid biosynthetic pathway in oleaginous fungus for dihomo-gamma linolenic acid production", JOURNAL OF BIOTECHNOLOGY, ELSEVIER, AMSTERDAM NL, vol. 218, 10 December 2015 (2015-12-10), pages 85-93, XP029379433, ISSN: 0168-1656, DOI: 10.1016/J.JBIOTEC.2015.12.003
- JAN GAJEWSKI ET AL: "Engineering fungal de novo fatty acid synthesis for short chain fatty acid production", NATURE COMMUNICATIONS, vol. 8, 10 March 2017 (2017-03-10), pages 1-8, XP055596556, DOI: 10.1038/ncomms14650

## Description

### TECHNICAL FIELD

The present invention relates to a recombinant mutant microorganism having the ability to produce malonic acid and a method for producing malonic acid using the same, and more particularly, to a mutant microorganism having the ability to produce malonic acid, wherein a semialdehyde dehydrogenase-encoding gene is introduced in a microorganism having the ability to produce malonic semialdehyde, and a method for producing malonic acid using the same.

### BACKGROUND ART

Malonic acid, a C3-dicarboxylic acid, is a chemical substance which is used in the production of various flavors, fragrances and pharmaceuticals. Malonic acid is in high demand, but conventional chemical methods for producing malonic acid have limitations due to high costs and environmentally hazardous production processes. Known methods of producing malonic acid via biological pathways include a method of producing malonic acid from malonyl-CoA by malonyl-CoA hydrolase activity (WO 2013134424).

NAKAMURA K ET AL:"Studies of malonic semialdehyde dehydrogenase from Pseudomonas aeruginosa", BIOCHIMICA ET BIOPHYSICA ACTA, ELSEVIER, NL, vol. 50, no. 1, 10 June 1961, (1961-06-10), pages 147-152 discloses a malonic semialdehyde dehydrogenase, which catalyses the diphosphopyridine nucleotide oxidation of malonic semialdehyde to malonate.

However, it is known that the malonyl-CoA pathway has a lower metabolite production efficiency than the aspartate pathway (Vinod Kumar et al., Biotechnology Advances 31:945-961, 2013; Valdehuesa et al., Appl. Microbiol. Biotechnol., 97:3309-3321, 2013). Thus, there is a need to develop a method of producing malonic acid in a more efficient manner than conventional chemical production methods and a method employing malonyl-CoA.

Therefore, it has been attempted to develop a method of producing malonic acid using malonic semialdehyde, which can be easily produced in cells, as a precursor by malonic semialdehyde dehydrogenase activity.

Accordingly, the present inventors have made extensive efforts to develop a method of more efficiently producing malonic acid via a biological pathway, and as a result, have produced a recombinant microorganism that expresses malonic semialdehyde dehydrogenase and beta alanine pyruvate transaminase and additionally expresses aspartate-α-decarboxylase, and have found that the use of the produced recombinant microorganism makes it possible to efficiently produce malonic acid from a carbon source, thereby completing the present invention.

### DISCLOSURE OF INVENTION

### TECHNICAL PROBLEM

It is an object of the present invention to provide a mutant microorganism of producing malonic acid using malonic semialdehyde as a precursor by malonic semialdehyde dehydrogenase activity.

Another object of the present invention is to provide a method of producing malonic acid using the mutant microorganism that utilizes a malonic semialdehyde pathway as a major malonic acid production pathway.

### TECHNICAL SOLUTION

To achieve the above object, the present invention provides a mutant microorganism as defined in claim 1. Preferred embodiments are reflected in the dependent claims.

The present invention also provides a method of producing malonic acid from sugar as defined in claim 10.

### BRIEF DESCRIPTION OF THE DRAWINGS

FIG. 1 shows the conversion of malonic semialdehyde to malonic acid by semialdehyde dehydrogenase.
FIG. 2 schematically shows the construction of a metabolic pathway and a strain, which produce malonic acid from glucose via beta alanine and malonic semialdehyde.

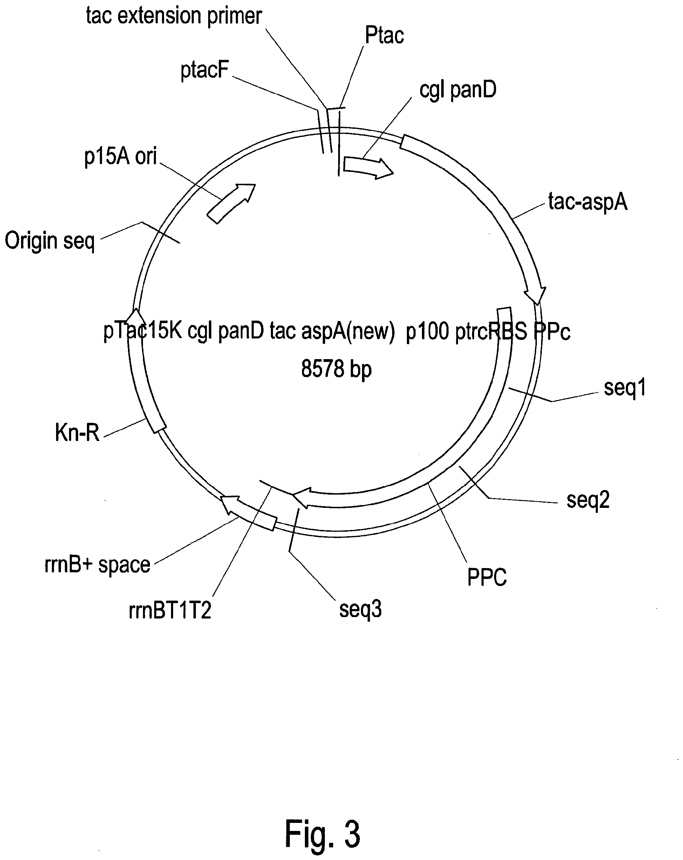

FIG. 3 shows a recombinant plasmid constructed by inserting *panD, aspA* and *ppc* genes to overproduce beta alanine.

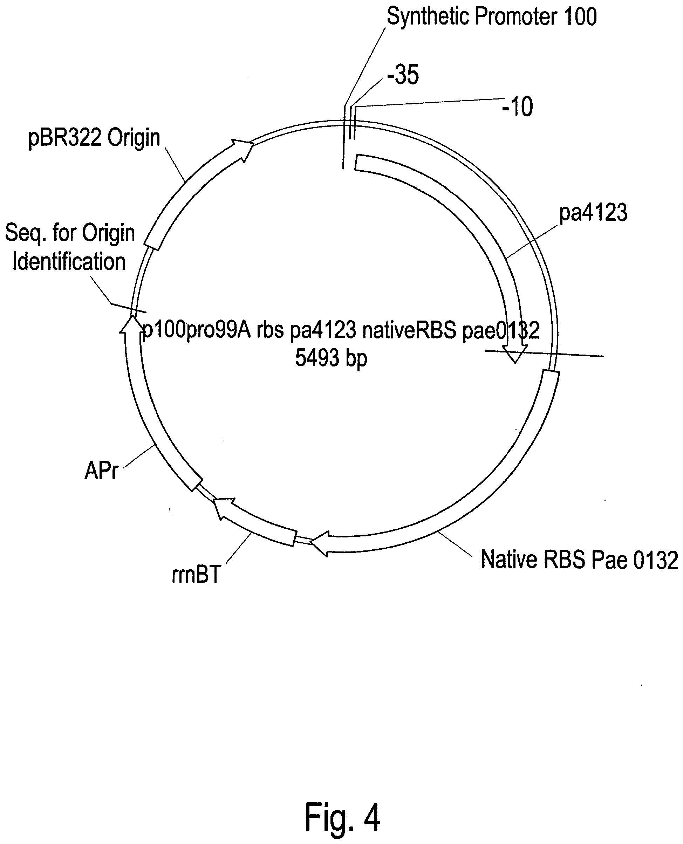

FIG. 4 shows a p100-99A pa4123pa0132 recombinant plasmid constructed by inserting *pa4123* gene and *pa0132* gene to produce malonic acid from beta alanine.

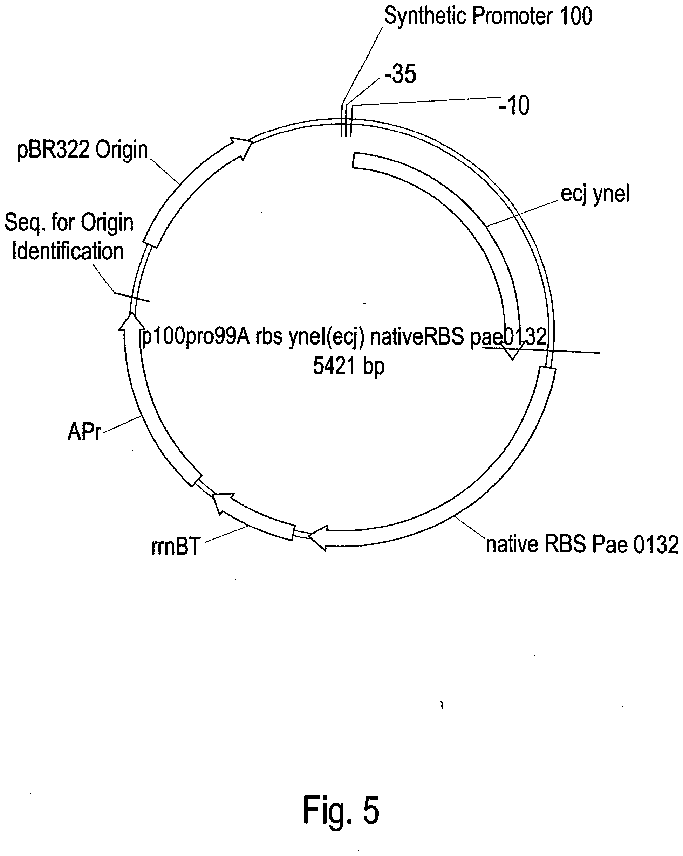

FIG. 5 shows a p100-99A yneIpa0132 recombinant plasmid constructed by inserting *yneI* gene and *pa0132* gene to produce malonic acid from beta alanine.

### BEST MODE FOR CARRYING OUT THE INVENTION

Unless defined otherwise, all the technical and scientific terms used herein have the same meaning as those generally understood by one of ordinary skill in the art to which the invention pertains. Generally, the nomenclature used herein and the experiment methods, which will be described below, are those well-known and commonly employed in the art.

In the present invention, a method of converting malonic semialdehyde to malonic acid by use of semialdehyde dehydrogenase was developed. In a specific example of the present invention, a previously developed beta-alanine-producing strain (Song et al., Metab. Eng., 30:121-129, 2015) was used, and a method which produces malonic acid was developed by producing malonic semialdehyde through expression of beta alanine pyruvate transaminase in the strain and additionally expressing semialdehyde dehydrogenase in the strain was developed.

In the present invention, a mutant microorganism having the ability to produce malonic acid from malonic semialdehyde by the semialdehyde dehydrogenase was prepared in which semialdehyde dehydrogenase represented by malonic semialdehyde dehydrogenase, succinic semialdehyde dehydrogenase or glutarate semialdehyde dehydrogenase is overexpressed.

Therefore, in one aspect, the present invention is directed to a mutant microorganism having the ability to produce malonic acid, wherein a semialdehyde dehydrogenase-encoding gene is introduced in a microorganism having the ability to produce malonic semialdehyde.

In the present invention, the semialdehyde dehydrogenase may be selected from the group consisting of malonic semialdehyde dehydrogenase, succinic semialdehyde dehydrogenase, and glutarate semialdehyde dehydrogenase. Preferably, the semialdehyde dehydrogenase is succinic semialdehyde dehydrogenase, but is not limited thereto.

In the present invention, the semialdehyde dehydrogenase-encoding gene may be either *pa4123* (SEQ ID NO: 10) or *yneI* (SEQ ID NO: 12), and is preferably a gene encoding enzyme having an amino acid sequence homology of at least 90% to PA4132 (SEQ ID NO: 11) or YneI (SEQ ID NO: 13). In addition, the gene *pa4123* of the present invention may be derived from *P*. *aeruginosa,* and the gene *ynel* of the present invention may be derived from *E.coli.*

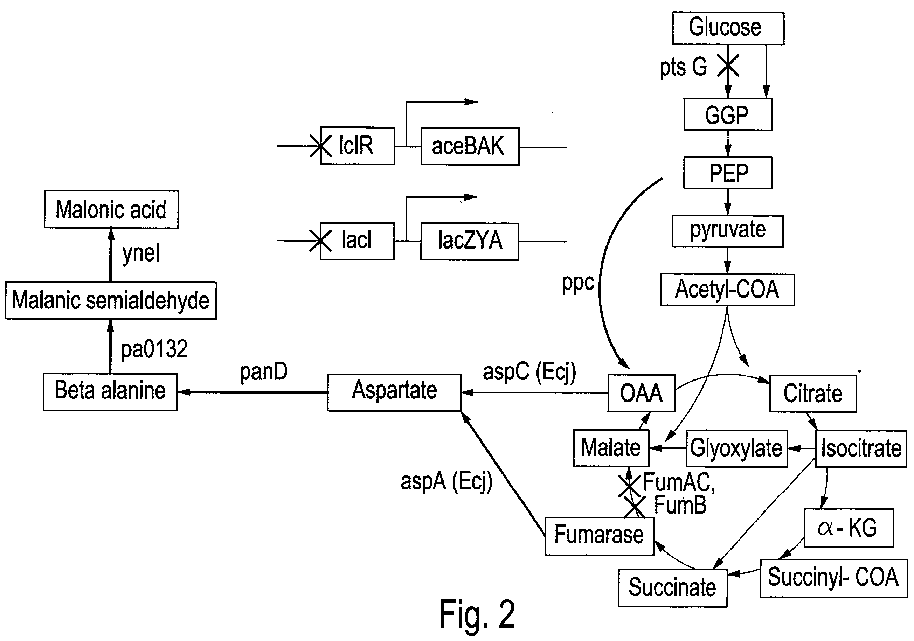

In the present invention, in order to make it possible to produce malonic acid from a carbon source, a previously developed parent strain which has an ability to produce beta alanine(FIG. 2) was used, and a system was established which can produce malonic acid from a carbon source by overexpressing beta alanine pyruvate transaminase and additionally overexpressing semialdehyde dehydrogenase to produce malonic semialdehyde from beta alanine.

In the present invention, preferably, one or more selected from the group consisting of a beta alanine pyruvate transaminase-encoding gene, an aspartate-α-decarboxylase-encoding gene, an aspartase-encoding gene, and a phosphoenolpyruvate carboxylase-encoding gene are additionally introduced, but is not limited thereto.

In the present invention, methods for increasing semialdehyde dehydrogenase activity include replacing a native promoter of a gene of interest on a genome with a stronger promoter *trc*, *tac, T7, lac, trp* or the like, and transforming a strain after cloning a semialdehyde dehydrogenase-encoding gene into an expression vector.

In the present invention, preferably, the aspartate-α-decarboxylase-encoding gene, the aspartase-encoding gene, and the phosphoenolpyruvate carboxylase-encoding gene are introduced in an expression vector containing a *trc* promoter, but are not limited thereto.

In the present invention, the beta alanine pyruvate transaminase-encoding gene may be *pa0132.*

In the present invention, the aspartate-α-decarboxylase-encoding gene may be *panD,* the aspartase-encoding gene may be *aspA,* and the phosphoenolpyruvate carboxylase-encoding gene may be *ppc.*

In the present invention, preferably, one or more genes selected from the group consisting of *ptsG* (phosphotransferase system), *fumC* (fumarase C), *fumA* (fumarase A), *fumB* (fumarase B), *iclR* (acetate operon repressor), and *lacI* (lac operon repressor) are additionally deleted, but is not limited thereto.

In the present invention, the microorganism having the ability to produce malonic semialdehyde is selected from the group consisting of bacteria, yeasts, and fungi. Preferably, the bacteria may be selected from the group consisting of *Zymomonas sp., Escherichia sp., Pseudomonas sp., Corynebacterium sp., Ralstonia sp., Klebsiella sp., Saccaromyces sp., Pichia sp., Salmonella sp., Kluyveromyces sp., Clostridium sp., Candida sp., Shigella sp., Burkholderia sp.,* and *Oligotropha sp.,* and may more preferably be selected from the group consisting of *Saccharomyces cerevisiae, Klebsiella pneumonia,* and *E*. *coli.*

In one example of the present invention, a previously developed beta-alanine-producing system (Song et al., Metab. Eng., 30:121-129, 2015) was used as a parent strain to produce a mutant microorganism that can produce malonic acid from a carbon source via malonic semialdehyde as an intermediate. A strain that can produce malonic acid was constructed through the overexpression of the beta alanine pyruvate transaminase-encoding gene *pa0132* (SEQ ID NO: 14) from *Pseudomonas aeruginosa* and the semialdehyde dehydrogenase-encoding gene *pa4123* (SEQ ID NO: 10) from *Pseudomonas aeruginosa.* In addition, a more efficient malonic acid production strain was constructed by overexpressing *ynel* (SEQ ID NO: 12) from *E. coli,* which is known as a succinic semialdehyde dehydrogenase-encoding gene instead of *pa4123.*

In another aspect, the present invention is directed to a mutant microorganism having the ability to produce malonic acid, wherein a beta-alanine-pyruvate transaminase-encoding gene and a semialdehyde dehydrogenase-encoding gene are introduced in a microorganism having the ability to produce beta-alanine.

In the present invention, preferably, the beta-alanine-pyruvate transaminase-encoding gene and the semialdehyde dehydrogenase-encoding gene are introduced in an expression vector containing a synthetic p100 promoter (SEQ ID NO: 9), but are not limited thereto.

In the present invention, the beta alanine pyruvate transaminase-encoding gene may be *pa0132* (SEQ ID NO: 14), and the semialdehyde dehydrogenase-encoding gene may be either *pa4123* or *ynel.*

In the present invention, preferably, one or more genes selected from the group consisting of a *panD* (aspartate-α-decarboxylase) gene, an *aspA* (aspartase A) gene, and a *ppc* (phosphoenol pyruvate carboxylase) gene are additionally introduced, and one or more genes selected from the group consisting of *ptsG* (phosphotransferase system), *fumC* (fumarase C), *fumA* (fumarase A), *fumB* (fumarase B), *iclR* (acetate operon repressor), and *lacI* (lac operon repressor) are additionally deleted, but is not limited thereto.

In still another aspect, the present invention is directed to a mutant microorganism having the ability to produce malonic acid, wherein an aspartate-α-decarboxylase-encoding gene, a beta-alanine-pyruvate transaminase-encoding gene, and a semialdehyde dehydrogenase-encoding gene are introduced in a microorganism having the ability to produce aspartate.

In the present invention, the aspartate-α-decarboxylase-encoding gene may be *panD,* the beta alanine pyruvate transaminase-encoding gene may be *pa0132,* and the semialdehyde dehydrogenase-encoding gene may be either *pa4123* or *ynel.*

In the present invention, preferably, an *aspA* gene or a *ppc* gene may be additionally introduced, and one or more genes selected from the group consisting of *ptsG, fumC, fumA, fumB, iclR,* and *lacI* may be additionally deleted, but the scope of the present invention is not limited thereto.

In the present invention, the microorganism having the ability to produce beta-alanine or aspartate may be selected from the group consisting of bacteria, yeasts, and fungi. Preferably, the bacteria may be selected from the group consisting of *Zymomonas sp., Escherichia sp., Pseudomonas sp., Corynebacterium sp., Ralstonia sp., Klebsiella sp., Saccharomyces sp., Pichia sp., Salmonella sp., Kluyveromyces sp., Clostridium sp., Candida sp., Shigella sp., Burkholderia sp.,* and *Oligotropha sp.,* and may more preferably be selected from the group consisting of *Saccharomyces cerevisiae, Klebsiella pneumonia,* and *E*. *coli.*

In yet another aspect, the present invention is directed to a method of producing malonic acid from sugar, the method comprising the steps of: culturing the above-described mutant microorganism in a medium containing a carbon source, thereby producing malonic acid; and recovering the produced malonic acid.

In the present invention, the carbon source may be selected from the group consisting of monosaccharides, disaccharides and polysaccharides, including glucose, sucrose, galactose, maltose, xylose, glycerol, fructose and sugar cane.

In an example of the present invention, only a certain medium and a certain culture method were illustrated. However, as reported in the literature (Lee et al., Bioprocess Biosyst. Eng., 26:63, 2003; Lee et al., Appl. Microbiol. Biotechnol., 58:663, 2002; Lee et al., Biotechnol. Lett., 25:111, 2003; Lee et al., Appl. Microbiol. Biotechnol., 54:23, 2000; Lee et al., Biotechnol. Bioeng., 72:41, 2001), the use of saccharification liquid such as whey or CSL (corn steep liquor), and other medium, or the use of various culture methods such as fed-batch culture or continuous culture, will also be obvious to a person of ordinary skill in the art.

In an example of the present invention, it could be seen that malonic acid was not produced in wild-type *E*. *coli* W3110 and beta-alanine production strain (Song et al., Metab. Eng., 30:121-129, 2015).

It could be seen that when a p100-99A pa4123pa0132 expression vector expressing the beta-alanine-pyruvate transaminase-encoding gene *pa0132* from *Pseudomonas aeruginosa* and the semialdehyde dehydrogenase-encoding gene *pa4123* from *Pseudomonas aeruginosa* were introduced in wild-type *E. coli* W3110, which was then cultured in a medium supplemented with 8 g/L of beta-alanine, 25 mg/L of malonic acid was produced.

From the above result, it could be seen that malonic acid could be produced from beta alanine through the overexpression of the beta alanine pyruvate transaminase and the semialdehyde dehydrogenase, which are well-known. In addition, it could be seen that when a p100-99A yneIpa0132 expression vector that expresses a succinic semialdehyde dehydrogenase-encoding *ynel* gene from *E. coli* instead of the semialdehyde dehydrogenase-encoding gene *pa4123* was introduced in wild-type *E. coli* W3110, which was then cultured in the medium, 60 m/L of malonic acid was produced.

From this result, it could be seen that when the p100-99A yneIpa0132 expression vector was introduced into a parent strain having the ability to produce beta-alanine and the strain was cultured in a medium supplemented with glucose, 200 mg/L of malonic acid could be produced from glucose.

As used herein, the term "deleting" is meant to include mutating, replacing (or substituting) or deleting part or all of the gene of interest, such that the gene is not expressed or does not exhibit enzymatic activity even though it is expressed, thereby blocking the biosynthetic pathway in which the gene is involved.

As used herein, the term "overexpression" refers to the expression of the gene of interest in cells at levels higher than the expression level of the gene under normal conditions, and is intended to include increasing expression levels either by replacing the promoter of a gene on the genome with a strong promoter or by cloning the gene of interest into an expression vector and transforming cells with the expression vector.

As used herein, the term "vector" means a DNA construct containing a DNA sequence operably linked to a suitable control sequence capable of effecting the expression of the DNA in a suitable host. The vector may be a plasmid, a phage particle, or simply a potential genomic insert. Once incorporated into a suitable host, the vector may replicate and function independently of the host genome, or may in some instances, integrate into the genome itself. In the present specification, "plasmid" and "vector" are sometimes used interchangeably, as the plasmid is the most commonly used form of vector. However, the present invention is intended to include other types of vectors with the same function as that would be known or known in the art. Typical expression vectors for mammalian cell culture expression are based on, for example, pRK5 (EP 307,247), pSV16B (WO91/08291), and pVL1392 (Pharmingen).

As used herein, the term "expression control sequence" refers to a DNA sequence essential for the expression of an operably linked coding sequence in a particular host organism. The control sequences include a promoter for performing transcription, a random operator sequence for controlling such transcription, a sequence for encoding a suitable mRNA ribosomal binding site (RBS), and a sequence for controlling the termination of transcription and translation. For example, a control sequence suitable for a prokaryotic cell includes a promoter, a random operator sequence, and an mRNA RBS. A control sequence suitable for a eukaryotic cell includes a promoter, a polyadenylation signal, and an enhancer.

The promoter is the most critical factor that affects the gene expression in a plasmid. Therefore, a high-expression promoter such as the SRα promoter or cytomegalovirus-derived promoter may be used.

To express the DNA sequence of the present invention, any of a wide variety of expression control sequences may be used in the vector. Examples of useful expression control sequences include the early and late promoters of SV40 or adenovirus as well as the lac system, the trp system, the TAC system, the TRC system, T3 and T7 promoters, the major operator and promoter regions of phage lambda, the control regions of fd coat protein, the promoter for 3-phosphoglycerate kinase or other glycolytic enzymes, the promoters of phosphatase, e.g., Pho5, the promoters of the yeast α-mating system, and other sequences with configurations or derivations known to control the expression of genes of prokaryotic or eukaryotic cells or their viruses, and various combinations thereof. T7 RNA polymerase promoter Φ10 may be effectively used to express the protein NSP in *E. coli.*

A nucleic acid is "operably linked" when it is placed in a functional relationship with another nucleic acid sequence. The nucleotide sequence may be a gene and a control sequence(s) linked to be capable of expressing the gene when proper molecules (e.g., transcription-activating protein) bind to a control sequence(s). For example, DNA for a pre-sequence or a secretory leader is operably linked to DNA encoding polypeptide when expressed as pre-protein participating in secretion of polypeptide; a promoter or an enhancer is operably linked to a coding sequence when affecting the transcription of the sequence; and a RBS is operably linked to a coding sequence when affecting the transcription of the sequence, or to a coding sequence when arranged to facilitate translation. Generally, the term "operably linked" means that the DNA linked sequences are contiguous, and in the case of the secretory leader, are contiguous and present in a reading frame. However, an enhancer is not necessarily contiguous. The linkage between these sequences is performed by ligation at a convenient restriction enzyme site. However, when the site does not exist, a synthetic oligonucleotide adaptor or a linker is used according to a conventional method.

The term "expression vector" as used herein generally means a double-stranded DNA fragment functioning as a recombinant carrier into which a heterologous DNA fragment is inserted. Here, the heterologous DNA means a hetero-type DNA, which is not naturally found in a host cell. The expression vector may be self-replicable regardless of host chromosomal DNA once in a host cell, and may produce several copies of the vector and (heterologous) DNA inserted thereinto.

As is well known in the art, in order to increase the expression level of a transfected gene in a host cell, a corresponding gene should be operably linked to transcription and translation expression control sequences which are operated in a selected expression host. Preferably, the expression control sequences and the corresponding gene are included in one expression vector together with a bacterial selection marker and a replication origin. When the expression host is a eukaryotic cell, the expression vector should further include a useful expression marker in a eukaryotic expression cell.

The host cell transformed or transfected by the aforementioned expression vector constitutes another aspect of the present invention. As used herein, the term "transformation" means that DNA can be replicated as a factor outside of chromosome or by means of completion of the entire chromosome by introducing DNA as a host. As used herein, the term "transfection" means that an expression vector is accepted by a host cell regardless of whether or not any coding sequence is actually expressed.

Host cells that are used in the present invention may be prokaryotic cells or eukaryotic cells. In addition, a host is generally used, into which DNA is introduced with high efficiency and in which the introduced DNA is expressed with high efficiency. Examples of host cells that can be used in the present invention include known prokaryotic and eukaryotic hosts such as *E. coli*, *Pseudomonas* spp., *Bacillus* spp., *Streptomyces* spp., fungi or yeast, insect cells such as *Spodoptera frugiperda* (SF9), animal cells such as CHO and mouse cells, African green monkey cells such as COS 1, COS 7, BSC 1, BSC 40 and BMT 10, and tissue-cultured human cells. In the present invention, when cDNA encoding the NSP protein is to be cloned, animal cells are preferably used as a host. When COS cells are used, a plasmid with SV40 replication origin can be present as multiple copies of an episome in the cells and can be expressed at higher levels than a conventional level, because the COS cell express SV40 large T antigen. The introduced DNA sequence may be obtained from the same species as the host cells, or may be of species different from the host cells, or may be a hybrid DNA sequence comprising any heterogeneous or homologous DNA.

Of course, it should be understood that all vectors and expression control sequences do not equally function to express DNA sequences according to the present invention. Similarly, all hosts do not equally function with respect to the same expression system. However, one skilled in the art may appropriately select from among various vectors, expression control sequences, and hosts without either departing from the scope of the present invention or bearing excessive experimental burden. For example, a vector must be selected considering a host cell, because the vector must be replicated in the host cell. Specifically, the copy number of the vector, the ability of regulating the copy number and the expression of other protein encoded by the corresponding vector (e.g., the expression of an antibiotic marker) should also be considered. Also, an expression control sequence may be selected taking several factors into consideration. For example, relative strength, control capacity and compatibility with the DNA sequence of the present invention of the sequence should be deliberated particularly with respect to possible secondary structures. Further, the selection of a host cell may be made under consideration of compatibility with a selected vector, toxicity of a product encoded by a DNA sequence, secretory nature of the product, ability to correctly fold a polypeptide, fermentation or cultivation requirements, ability to ensure easy purification of a product encoded by a DNA sequence, or the like. Within the scope of these parameters, one of ordinary skill in the art may select various vectors / expression control sequences / host combinations that can express the DNA sequences of the invention in either large scale animal culture or fermentation. In cloning the cDNA of an NSP protein by the expression cloning strategy, screening procedures such as a binding method, a panning method, and a film emulsion method can be used.

In the definition of the present invention, the term "substantially pure" means that a polypeptide according to the present invention and the DNA sequences encoding the polypeptide substantially do not contain any other proteins derived from bacteria.

As host cells for expressing recombinant proteins, prokaryotic cells, such as *E. coli* and *Bacillus subtilis,* which can be cultured at a high concentration within a short time, easily genetically modified and have well established genetic and physiological properties, have been widely used. However, to solve various problems, including the post-translational modification, secretion, three-dimensional active structure and activation of proteins, a wide range from microorganisms to higher organisms, including unicellular eukaryotic cells, yeasts *(Pichia pastoris, Saccharomyces cerevisiae, Hansenula polymorpha,* etc.), filamentous fungi, insect cells, plant cells, and mammalian cells, has recently been used as host cells for recombinant protein production. Thus, it will be obvious to one skilled in the art to use not only *E. coli* cells illustrated in Examples, but also other host cells.

### EXAMPLES

Hereinafter, the present invention will be described in further detail with reference to examples. It will be obvious to a person having ordinary skill in the art that these examples are for illustrative purposes only and are not to be construed to limit the scope of the present invention.

In particular, *E. coli* W3110 was used as a host microorganism in the following examples. However, it will be obvious to those skilled in the art that other *E. coli* strains, bacteria, yeasts or fungi may also be used. In addition, in the following examples, only genes from specific strains were illustrated as the genes to be introduced; however, it will be obvious to those skilled in the art that the genes to be introduced are not limited as long as they are expressed in a host cell into which they are introduced, and exhibit the same activity as the illustrated genes.

### Example 1: Construction of Vector

### 1-1: pTac15k PTAP Vector for Producing Beta-Alanine from Carbon Source

Using the chromosomal DNA of *E. coli* W3100 as a template, PCR was performed with primers of the following SEQ ID NOs: 1 and 2, thereby constructing a phosphoenolpyruvate carboxylase-encoding *ppc* gene fragment.
[SEQ ID NO: 1] ppc F
   5'-AGACAGCCTGCAGGTTAGCCGGTATTACGCATACCTG-3'
[SEQ ID NO: 2] ppc R
   5'-AGACAGCCTGCAGGACAGGAAACAGACCATGAACGAACAATATTCCGC-3'.

Next, the constructed *ppc* fragment was treated with *Sbf*I restriction enzyme, and the plasmid pTac15k PTA (Song et al., Metab. Eng., 30:121-129, 2015), which expresses an aspartate-α-decarboxylase-encoding *panD* gene derived from a *Corynebacterium glutamicum* strain (ATCC 13032) and an aspartase-encoding *aspA* gene derived from *E. coli* W3110, was treated with *SbfI* restriction enzyme. Thereafter, the *ppc* fragment and the pTac15k PTA were ligated to each other by T4 DNA ligase, thereby constructing the recombinant plasmid pTac15k PTAP.

### 1-2: p100-99A pa4123pa0132 and p100-99A yneIpa0132 Vectors for Producing Malonic Acid from Beta-Alanine

Using the chromosomal DNA of a *Pseudomonas aeruginosa* PA01 strain as a template, PCR was performed with primes of the following SEQ ID NOs: 3 and 4, thereby constructing a beta-alanine-pyruvate transaminase-encoding *pa0132* gene fragment. Similarly, using the chromosomal DNA of a *Pseudomonas aeruginosa* PA01 strain as a template, PCR was performed with primes of the following SEQ ID NOs: 5 and 6, thereby constructing a semialdehyde dehydrogenase-encoding *pa4123* gene fragment. In addition, using the chromosomal DNA of an *E. coli* W3110 strain as a template, PCR was performed with primes of the following SEQ ID NOs: 7 and 8, thereby constructing a succinic semialdehyde dehydrogenase-encoding *ynel* gene fragment:
[SEQ ID NO: 3] pa0132 F
   5'-AGACAGTCTAGAGAAAGCCCGAGGATCGAACGA-3'
[SEQ ID NO: 4] pa0132 R
   5'-AGACAGCCTGCAGGTCAGGCGATGCCGTTGAGC-3'
[SEQ ID NO: 5] pa4123 F 
5'-
AGACAGGAGCTCACAGGAAACAGACCATGATCAAACACTGGATCAATGGCC-3'
[SEQ ID NO: 6] pa4123 R
   5'-AGACAGTCTAGACTACACGCCCCAGCGGGGG-3'
[SEQ ID NO: 7] yneI F 
5'-
AGACAGGAGCTCACAGGAAACAGACCATGACCATTACTCCGGCAACTCATG-3'
[SEQ ID NO: 8] yneI R
   5'-AGACAGTCTAGATCAGATCCGGTCTTTCCACACCG-3'.

Next, the constructed *pa0132* fragment was treated with the restriction enzymes *XbaI* and *SbfI* in order to ligate it to a p100-99A plasmid that regulates gene expression by a p100 promoter (SEQ ID NO. 9: ttgacggctagctcagtcctaggtacagtgctagc). Thereafter, based on this vector, *pa4123* and *yneI* fragments were treated with *SacI* and *XbaI* and ligated to the vector by T4 DNA ligase, thereby constructing the recombinant plasmids p100-99A pa4123pa0132 and p100-99A yneIpa0132.

### Example 2: Measurement of the Malonic Acid-Producing Ability of Recombinant Mutant Microorganism

### 2-1: Mutant Microorganism Producing Malonic Acid from Beta-Alanine

The p100-99A pa4123pa0132 or p100-99A yneIpa0132 plasmid constructed in Example 1-2 was introduced into an *E. coli* W3110 strain, which was then cultured in a medium supplemented with 8 g/L of beta-alanine, and the production of malonic acid in the strain was analyzed (Table 1).

A recombinant mutant microorganism having malonic acid-producing ability as a result of introducing therein the vector for producing malonic acid from beta-alanine was selected on an LB plate medium supplemented with 30 ug/ml of kanamycin and 30 ug/ml of ampicillin. The transformed microorganism was inoculated into 10 ml LB medium and precultured at 37°C for 12 hours, and then 3 ml of the preculture was inoculated and cultured in 50 ml of modified MR medium in a 350-ml flask.

The composition of the modified MR medium (pH 6.5) was composed of, per liter of distilled water, 15 g of glucose, 9 g of (NH₄)₂SO₄, 6.67 g of KH₂PO₄, 4.0 g of (NH₄)₂HPO₄, 3.0 g of yeast extract, 2.0 g of NaHCO₃, 0.8 g of citric acid, 0.8 g of MgSO₄7H₂O, 0.01 g of CaCl₂2H₂0, and 5 ml of a trace metal solution (composed of, per liter of distilled water, 10 g of FeSO₄7H₂O, 2.2g of ZnSO₄4H₂O, 0.58g of MnSO₄4H₂O, 1.0 g of CuSO₄5H₂O, 0.1g of (NH₄)6Mo₇O₂₄4H₂O, and 0.02g of Na₂B₄O₇10H₂O). The culturing was performed in a shaking incubator at 220 rpm at 37°C for 24 hours. After completion of the culturing, the culture was centrifuged at 10,000 rpm for 10 minutes, and the supernatant was collected and analyzed by liquid chromatography to measure the production of malonic acid.

As a result, as shown in Table 1 below, it could be seen that wild-type *E. coli* W3110 produced no malonic acid, and the transformed microorganisms introduced with the p100-99A pa4123pa0132 or p100-99A yneIpa0132 plasmid produced malonic acid in amounts of 25 mg/L and 60 mg/L, respectively, by addition of beta-alanine.

**Table 1: Amount of malonic acid produced by addition of beta-alanine**

| Strain | Malonic acid (mg/L) |
|---|---|
| W3110 | 0 |
| W3110 + p100-99A pa4123pa0132 | 25 |
| W3110 + p100-99A yneIpa0132 | 60 |

### 2-2: Mutant Microorganism Producing Malonic Acid from Glucose

Next, the pTac15k PTAP plasmid, constructed in Example 1-1, and the p100-99A yneIpa0132 plasmid constructed in Example 1-2 were introduced into CWF4NA (Song et al., Metab. Eng., 30:121-129, 2015), an aspartic acid-producing strain in which *iclR, fumC, fumA, fumB, ptsG* and *lacI* genes are deleted and in which the promoter of the *aspA* gene is substituted with a strong *trc* promoter, thereby constructing a recombinant mutant microorganism. As controls, the *E. coli* W3110 strain and the CWF4NA strain not introduced with the pTac15k PTAP and p100-99A yneIpa0132 vectors were used. The production of malonic acid from glucose by the recombinant mutant organism was analyzed (Table 2).

A recombinant mutant microorganism having malonic acid-producing ability from glucose was selected on an LB plate medium supplemented with 30 ug/ml of kanamycin and 30 ug/ml of ampicillin. The transformed strain was inoculated into 10 ml LB medium and precultured at 37°C for 12 hours, and then 3 ml of the preculture was inoculated and cultured in 50 ml of modified MR medium in a 350-ml flask.

The composition of the modified MR medium (pH 6.5) was composed of, per liter of distilled water, 15 g of glucose, 9 g of (NH₄)₂SO₄, 6.67 g of KH₂PO₄, 4.0 g of (NH₄)₂HPO₄, 3.0 g of yeast extract, 2.0 g of NaHCO₃, 0.8 g of citric acid, 0.8 g of MgSO₄7H₂O, 0.01 g of CaCl₂2H₂0, and 5 ml of a trace metal solution (composed of, per liter of distilled water, 10.0 g of FeSO₄7H₂O, 2.2g of ZnSO₄4H₂O, 0.58g of MnSO₄4H₂O, 1.0 g of CuSO₄5H₂O, 0.1g of (NH₄) 6Mo₇O₂₄4H₂O, and 0.02g of Na₂B₄O₇10H₂O). The culturing was performed in a shaking incubator at 220 rpm at 37°C for 24 hours. After completion of the culturing, the culture was centrifuged at 10,000 rpm for 10 minutes, and the supernatant was collected and analyzed by liquid chromatography to measure the production of malonic acid.

As a result, as shown in Table 2 below, it could be seen that when pTac15k PTAP and p100-99A yneIpa0132 are introduced into a CWF4NA strain, about 200 mg/L of malonic acid was produced from glucose. In other words, this result suggests that malonic acid can be produced from malonic semialdehyde by the expression of semialdehyde dehydrogenase.

**Table 2: Amount (mg/L) of Malonic Acid Produced by Recombinant Mutant Microorganism after Addition of Glucose**

| Strain | Malonic acid (mg/L) |
|---|---|
| W3110 | 0 |
| CWF4NA | 0 |
| CWF4NA + pTac15k PTAP + p100-99A yneIpa0132 | 200 |

### INDUSTRIAL APPLICABILITY

The mutant microorganism according to the present invention is useful for effectively producing malonic acid from beta-alanine or various carbon sources via malonic semialdehyde as an intermediate metabolite through expression of an enzyme having semialdehyde dehydrogenase activity by a biological method.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims.

## Claims

1. A mutant microorganism having the ability to produce malonic acid, wherein a semialdehyde dehydrogenase-encoding gene is introduced in a microorganism having the ability to produce malonic semialdehyde, wherein the semialdehyde dehydrogenase-encoding gene is either *pa4123* according to SEQ ID NO: 10 or *yneI* according to SEQ ID NO: 12 and said microorganism is selected from the group consisting of bacteria, yeasts, and fungi.

2. The mutant microorganism of claim 1, wherein one or more selected from the group consisting of a beta alanine pyruvate transaminase-encoding gene, an aspartate-α-decarboxylase-encoding gene, an aspartase-encoding gene, and a phosphoenolpyruvate carboxylase-encoding gene are additionally introduced, wherein the beta alanine pyruvate transaminase-encoding gene is *pa0132* according to SEQ ID NO: 14; or wherein the aspartate-α-decarboxylase-encoding gene is *panD;* or wherein the aspartase-encoding gene is *aspA;* or wherein the phosphoenolpyruvate carboxylase-encoding gene is *ppc.*

3. The mutant microorganism of claim 2, wherein the aspartate-α-decarboxylase-encoding gene, the aspartase-encoding gene, and the phosphoenolpyruvate carboxylase-encoding gene are introduced in an expression vector containing a trc promoter.

4. The mutant microorganism of claim 1, wherein one or more genes selected from the group consisting of *ptsG, fumC, fumA, fumB, iclR,* and *lacI* are additionally deleted.

5. The mutant microorganism of claim 1, wherein a beta alanine pyruvate transaminase-encoding gene is additionally introduced, wherein the beta alanine pyruvate transaminase-encoding gene is *pa0132* according to SEQ ID NO: 14.

6. The mutant microorganism of claim 5, wherein the beta alanine pyruvate transaminase-encoding gene and the semialdehyde dehydrogenase-encoding gene are introduced in an expression vector containing a synthetic p100 promoter according to SEQ ID NO: 9.

7. The mutant microorganism of claim 6, wherein one or more genes selected from the group consisting of a *panD* gene, an *aspA* gene, and a *ppc* gene are additionally introduced, and one or more genes selected from the group consisting of *ptsG, fumC, fumA, fumB, iclR,* and *lacI* are additionally deleted.

8. The mutant microorganism of claim 5, wherein an aspartate-α-decarboxylase-encoding gene is additionally introduced, wherein the aspartate-α-decarboxylase-encoding gene is *panD.*

9. The mutant microorganism of claim 8, wherein an *aspA* gene or a *ppc* gene are additionally introduced, and one or more genes selected from the group consisting of *ptsG, fumC, fumA, fumB, iclR,* and *lacI* are additionally deleted.

10. A method of producing malonic acid from sugar, the method comprising the steps of: producing malonic acid by culturing the mutant microorganism of any one of claims 1 to 9 in a medium containing a carbon source; and recovering the produced malonic acid, wherein preferably the carbon source is selected from the group consisting of glucose, sucrose, galactose, maltose, xylose, glycerol, fructose, sugar cane and beta alanine.

## Patentansprüche

1. Mutanter Mikroorganismus mit der Fähigkeit, Malonsäure zu produzieren, wobei ein Semialdehyd-Dehydrogenase codierendes Gen in einen Mikroorganismus mit der Fähigkeit, Malonat-Semialdehyd zu produzieren, eingeführt ist, wobei es sich bei dem Semialdehyd-Dehydrogenase codierenden Gen entweder um *pa4123* gemäß SEQ ID Nr. 10 oder um *yneI* gemäß SEQ ID Nr. 12 handelt und der Mikroorganismus aus der Gruppe ausgewählt ist, die aus Bakterien, Hefen und Pilzen besteht.

2. Mutanter Mikroorganismus gemäß Anspruch 1, wobei eines oder mehrere, die aus der Gruppe ausgewählt sind, die aus einem beta-Alanin-Pyruvat-Transaminase codierenden Gen, einem Aspartat-o-Decarboxylase codierenden Gen, einem Aspartase codierenden Gen und einem Phosphoenolpyruvat-Carboxylase codierenden Gen besteht, zusätzlich eingeführt sind, wobei es sich bei dem beta-Alanin-Pyruvat-Transaminase codierenden Gen um *pa0132* gemäß SEQ ID Nr. 14 handelt; oder wobei es sich bei dem Aspartat-o-Decarboxylase codierenden Gen um *panD* handelt; oder wobei es sich bei dem Aspartase codierenden Gen um *aspA* handelt; oder wobei es sich bei dem Phosphoenolpyruvat-Carboxylase codierenden Gen um *ppc* handelt.

3. Mutanter Mikroorganismus gemäß Anspruch 2, wobei das Aspartat-α-Decarboxylase codierende Gen, das Aspartase codierende Gen und das Phosphoenolpyruvat-Carboxylase codierende Gen in einen Expressionsvektor, der einen *trc*-Promotor enthält, eingeführt sind.

4. Mutanter Mikroorganismus gemäß Anspruch 1, wobei ein oder mehrere Gene, die aus der Gruppe ausgewählt sind, die aus *ptsG, fumC, fumA, fumB, iclR* und *lacI* besteht, zusätzlich deletiert sind.

5. Mutanter Mikroorganismus gemäß Anspruch 1, wobei zusätzlich ein beta-Alanin-Pyruvat-Transaminase codierendes Gen eingeführt ist, wobei es sich bei dem beta-Alanin-Pyruvat-Transaminase codierenden Gen um *pa0132* gemäß SEQ ID Nr. 14 handelt.

6. Mutanter Mikroorganismus gemäß Anspruch 5, wobei das beta-Alanin-Pyruvat-Transaminase codierende Gen und das Semialdehyd-Dehydrogenase codierende Gen in einen Expressionsvektor eingeführt sind, der einen synthetischen p100-Promotor gemäß SEQ ID Nr. 9 enthält.

7. Mutanter Mikroorganismus gemäß Anspruch 6, wobei ein oder mehrere Gene, die aus der Gruppe ausgewählt sind, die aus einem *panD-*Gen, einem *aspA-*Gen und einem *ppc*-Gen besteht, zusätzlich eingeführt sind und ein oder mehrere Gene, die aus der Gruppe ausgewählt sind, die aus *ptsG, fumC, fumA, fumB, iclP* und *lacI* besteht, zusätzlich deletiert sind.

8. Mutanter Mikroorganismus gemäß Anspruch 5, wobei ein Aspartat-α-Decarboxylase codierendes Gen zusätzlich eingeführt ist, wobei es sich bei dem Aspartat-o-Decarboxylase codierenden Gen um *panD* handelt.

9. Mutanter Mikroorganismus gemäß Anspruch 8, wobei ein ein *aspA-*Gen oder ein ppc-Gen zusätzlich eingeführt sind und ein oder mehrere Gene, die aus der Gruppe ausgewählt sind, die aus *ptsG, fumC, fumA, fumB, iclR* und *lacI* besteht, zusätzlich deletiert sind.

10. Verfahren zur Herstellung von Malonsäure aus Zucker, wobei das Verfahren die folgenden Schritte umfasst: Produzieren von Malonsäure durch Kultivieren des mutanten Mikroorganismus gemäß einem der Ansprüche 1 bis 9 in einem Medium, das eine Kohlenstoffquelle enthält; und Gewinnen der produzierten Malonsäure, wobei vorzugsweise die Kohlenstoffquelle aus der Gruppe ausgewählt ist, die aus Glucose, Saccharose, Galactose, Maltose, Xylose, Glycerin, Fructose, Zuckerrohr und beta-Alanin besteht.

## Revendications

1. Microorganisme mutant ayant la capacité de produire de l'acide malonique, dans lequel un gène codant pour semialdéhyde deshydrogénase est introduit dans un microorganisme ayant la capacité de produire du semialdéhyde malonique, dans lequel ledit gène codant pour semialdéhyde deshydrogénase est soit *pa4123* selon SEQ ID n° 10 ou *yneI selon* SEQ ID n° 12, et ledit microorganisme est choisi dans le groupe consistant en bactéries, levures, et champignons.

2. Microorganisme mutant selon la revendication 1, dans lequel l'un ou plusieurs choisis dans le groupe consistant en un gène codant pour β-alanine/pyruvate transaminase, un gène codant pour aspartate-α-décarboxylase, un gène codant pour aspartase, et un gène codant pour phosphoénolpyruvate carboxylase sont introduits en outre, dans lequel ledit gène codant pour β-alanine/pyruvate transaminase est *pa0132* selon SEQ ID n° 14, ou dans lequel ledit gène codant pour aspartate-α-décarboxylase est *panD,* ou dans lequel ledit gène codant pour aspartase est aspA, ou dans lequel ledit gène codant pour phosphoénolpyruvate carboxylase est *ppc.*

3. Microorganisme mutant selon la revendication 2, dans lequel ledit gène codant pour aspartate-α-décarboxylase, ledit gène codant pour aspartase, et ledit gène codant pour phosphoénolpyruvate carboxylase sont introduits dans un vecteur d'expression contenant un promoteur *trc.*

4. Microorganisme mutant selon la revendication 1, dans lequel un ou plusieurs gènes choisis dans le groupe consistant en *ptsG, fumC, fumA, fumB, iclR,* et *lacI* sont délétés en outre.

5. Microorganisme mutant selon la revendication 1, dans lequel un gène codant pour β-alanine/pyruvate transaminase est introduit en outre, dans lequel ledit gène codant pour β-alanine/pyruvate transaminase est *pa0132* selon SEQ ID n° 14.

6. Microorganisme mutant selon la revendication 5, dans lequel ledit gène codant pour β-alanine/pyruvate transaminase et ledit gène codant pour semialdéhyde deshydrogénase sont introduits dans un vecteur d'expression contenant un promoteur p100 synthétique selon SEQ ID n° 9.

7. Microorganisme mutant selon la revendication 6, dans lequel un ou plusieurs gènes choisis dans le groupe consistant en un gène *panD,* un gène *aspA,* et un gène *ppc* sont introduits en outre, et un ou plusieurs gènes choisis dans le groupe consistant en *ptsG, fumC, fumA, fumB, iclR,* et *lacI* sont délétés en outre.

8. Microorganisme mutant selon la revendication 5, dans lequel un gène codant pour aspartate-α-décarboxylase est introduit en outre, dans lequel ledit gène codant pour aspartate-α-décarboxylase est *panD.*

9. Microorganisme mutant selon la revendication 8, dans lequel un gène *aspA* ou un gène *ppc* sont introduits en outre, et un ou plusieurs gènes choisis dans le groupe consistant en *ptsG, fumC, fumA, fumB, iclR,* et *lacI* sont délétés en outre.

10. Procédé pour produire de l'acide malonique à partir de sucre, ledit procédé comprenant les étapes consistant à produire de l'acide malonique en cultivant ledit microorganisme mutant selon l'une quelconque des revendications 1 à 9 dans un milieu contenant une source de carbone, et récupérer l'acide malonique produit, dans lequel, de préférence, ladite source de carbone est choisie dans le groupe consistant en glucose, saccharose, galactose, maltose, xylose, glycérol, fructose, canne, et béta-alanine.
